# FASCICULE DE BREVET EUROPEEN

(11) **EP 0 912 653 B1**
(45) Date de publication et mention de la délivrance du brevet: **14.11.2001**
(21) Numéro de dépôt: 97933729.2
(22) Date de dépôt: 11.07.1997
(51) Int. Cl.: C09K 7/02

(54) **FLUIDE COMPRENANT DES NANOFIBRILLES DE CELLULOSE ET SON APPLICATION POUR L'EXPLOITATION DE GISEMENTS PETROLIERS**
CELLULOSENANOFIBRILLEN ENTHALTENDE FLÜSSIGKEIT UND SEINE VERWENDUNG ZUR BETREIBUNG VON ERDÖLFELDERN
FLUID COMPRISING CELLULOSE NANOFIBRILS AND ITS USE FOR OIL MINING

(30) Priorité: 15.07.1996 FR 9609062; 15.07.1996 FR 9609061; 02.08.1996 FR 9609944; 27.09.1996 FR 9611986; 27.09.1996 FR 9611779
(43) Date de publication de la demande: 06.05.1999
(73) Titulaire: RHODIA CHIMIE, 92408 Courbevoie Cédex (FR)
(72) Inventeur: LANGLOIS, Bruno, F-91700 Saint Genevieve des Bois (FR); GUERIN, Gilles, 95600 Eaubonne (FR); SENECHAL, Alain, 94220 Charenton (FR); CANTIANI, Robert, 92800 Puteaux (FR); VINCENT, Isabelle, 27000 Evreux (FR); BENCHIMOL, Joel, 27800 Francqueville (FR)
(74) Mandataire: Dubruc, Philippe
(86) Numéro de dépôt international: FR9701297
(87) Numéro de publication internationale: WO9802499

(56) Documents cités:
- EP-A- 0 051 230
- EP-A- 0 726 356
- WO-A-85/00402
- WO-A-89/08148
- WO-A-92/22621
- WO-A-93/18111

## Description

La présente invention a trait aux fluides mis en oeuvre en présence d'eau, dont les applications se trouvent dans le domaine de l'extraction du pétrole, que ce soit pour les opérations de mise en place du puits, avec les opérations de forage, les opérations dites de "work-over", de complétion, que de l'exploitation proprement dite du gisement pétrolifère.

Les opérations de forage consistent à creuser un trou au moyen d'un trépan en carbure de tungstène notamment, fixé à des tiges creuses vissées bout à bout. Le plus souvent, de la boue comprenant des additifs en solution aqueuse est injectée dans le train de tiges. Cette boue remonte ensuite par le trou de sonde, extérieurement aux tiges, et entraîne des éléments de roches détachés lors de l'opération de forage. Dans le même temps, la boue chargée des roches établit une contre pression qui consolide le trou. La boue est ensuite extraite du trou de forage pour être débarrassée des roches qu'elle contient avant d'être injectée à nouveau dans les tiges creuses de forage.

Dans de telles conditions de mise en oeuvre, il est essentiel que les additifs ajoutés à la boue confèrent à celle-ci un comportement rhéologique particulier. En effet, lorsqu'il est soumis à de très fortes contraintes de cisaillement et des températures élevées, ainsi que c'est le cas au niveau du trépan, le fluide doit avoir une viscosité suffisamment faible pour faciliter son évacuation vers l'extérieur des tiges creuses. Par contre, ce même fluide, chargé des roches, doit présenter une viscosité élevée afin de maintenir en suspension les déblais entraînés lors du forage.

L'utilisation de polysaccharides à haut poids moléculaire, tels que la gomme xanthane par exemple, comme additif des boues ou fluides de forage, est bien connue afin de conférer au dit fluide le type de comportement rhéologique particulier décrit ci-dessus, et appelé rhéofluidifiant.

Cependant, si la gomme xanthane présente des avantages indéniables dans ce type d'application, ils restent néanmoins limités car les propriétés rhéologiques du fluide de forage se dégradent au cours du temps et ceci d'autant plus rapidement que la température à laquelle il est soumis avoisine ou dépasse 120°C. Or dans les opérations de forage pétrolier, c'est bien souvent que de telles températures sont atteintes. Tout d'abord, l'échauffement de la boue provoqué par le mouvement du trépan est important. De plus, la profondeur du gisement et sa situation géographique (puits chaud) ont aussi des conséquences sur la température à laquelle est soumis le fluide. Ainsi on peut atteindre sans difficulté des températures au fond du puits de cet ordre de grandeur car la profondeur atteinte lors des opérations de forage est de l'ordre du kilomètre voire supérieure. En outre il existe des puits pour lesquels la température de la croûte terrestre présente déjà une température plus élevée qu'ailleurs (puits chauds), ce qui accentue encore l'effet de l'augmentation de la température du sous-sol avec la profondeur.
En cycle normal, l'effet provoqué par de telles températures n'est pas systématiquement rédhibitoire, en tout cas en début d'utilisation de l'additif, car la durée pendant laquelle le fluide se trouve à ces fortes températures est limitée. Cependant, il faut savoir qu'il n'est pas rare de devoir arrêter les opérations de forage pour effectuer un remplacement de l'un des outils, le trépan par exemple. Or dans un tel cas, le fluide de forage reste pendant une durée relativement longue dans des conditions de température importante.

Lorsque le fluide est soumis à des températures élevées, la viscosité de ce dernier se dégrade considérablement et bien souvent le rôle de suspension des déblais arrachés lors de l'opération de forage n'est plus maintenu. Ceci peut donc être l'une des causes d'un colmatage du puits par dépôt des roches au fond de ce dernier.

La demande de brevet européen EP 134 084 décrit l'utilisation de microfibrilles de cellulose comme additif pour fluide de forage pétrolier. Cependant, si la résistance en température de cet additif est meilleure que celle de la gomme xanthane, elle reste néanmoins limitée à 160°C ; lorsqu'elle est utilisée comme seul viscosifiant.

La présente invention a donc pour but de proposer un additif pour fluide de forage présentant une meilleure stabilité thermique que les polysaccharides employés, une meilleure stabilité thermique que les microfibrilles de cellulose dont il a été question ci-dessus, tout en conservant les avantages de ces derniers.

Ainsi, la présente invention a pour objet de proposer un fluide comprenant un composé susceptible de conférer au dit fluide un comportement à l'écoulement bien spécifique.

De plus, l'additif selon l'invention, du fait de sa stabilité améliorée à la température, permet de conserver ces propriétés rhéologiques au fluide, même à des températures très élevées, c'est-à-dire au moins égales à 180°C, voire jusqu'à 200 °C.

Par ailleurs, l'additif selon l'invention est stable en milieu aqueux comprenant des sels ou tout autre type d'additifs employés couramment dans ce type d'application pour contrôler ou adapter les caractéristiques du fluide, tels que les capteurs d'oxygène, les réducteurs de filtrat, des modificateurs de rhéologie, tels que les charges, les alourdissants solubles ou non.

En outre, l'additif utilisé selon l'invention présente la caractéristique de conserver ses propriétés particulières viscosantes dans une gamme de composition très entendue d'eau, le rendant approprié pour des utilisations en présence d'eaux très minéralisées ou d'eaux de mer.

Le fluide selon l'invention est de plus tout à fait approprié, de par ses propriétés stables rhéofluidifiantes, pour des utilisations proches de la zone de production du pétrole, ou lors de forages déviés dans lesquels, pour des raisons d'écoulement et d'endommagement de la formation, les fluides doivent de préférence être formulés avec peu ou pas de solides.

Un avantage supplémentaire de l'additif selon l'invention est qu'il présente une filtrabilité meilleure que les polysaccharides du type de la gomme xanthane, ce qui diminue les risques d'endommagement du site de forage en évitant la formation de bouchon dans le puits. Par ailleurs, cela peut contribuer à faciliter le traitement ultérieur des boues de forage, soit la séparation des déblais, avant le recyclage des boues dans le puits.

Ces buts et d'autres sont atteints par la présente invention qui consiste en un fluide de forage pétrolier comprenant des nanofibrilles de cellulose présentant au moins environ 80 % de cellules à parois primaires et chargées en acides carboxyliques, en polysaccharides acides, seuls ou en mélange.

Mais d'autres avantages et caractéristiques de la présente invention apparaîtront plus clairement à la lecture de la description et des exemples qui vont suivre.

Avant de décrire le fluide selon l'invention, il faut préciser que ce dernier est particulièrement approprié pour être utilisé en tant que fluide de forage.

Cependant ses propriétés rhéologiques, sa filtrabilité, sa compatibilité avec de nombreux composants, le rendent tout aussi convenable pour des applications ultérieures au forage proprement dit et ou des applications liées à l'exploitation même du gisement.

Ainsi, moyennant une adaptation de ses caractéristiques, comme notamment la viscosité, le fluide peut être employé pendant les opérations dites de "work-over". Il est de même possible d'utiliser le fluide, toujours après adaptation de ses caractéristiques rhéologiques, pour la récupération assistée du pétrole.

Pour des raisons de simplification, dans la suite du texte ne sera mentionnée que l'application du fluide selon l'invention dans les opérations de forage, sachant que l'utilisation d'un tel fluide n'est pas limitée à cette seule application.

Ainsi que cela a été indiqué ci-dessus, le fluide de forage selon la présente invention comprend en tant qu'additif des nanofibrilles de cellulose bien spécifiques qui présentent, contrairement aux microfibrilles classiques, telles que celles décrites notamment dans la demande de brevet européen précitée, des avantages particulièrement intéressants et surprenants.

Ainsi, les nanofibrilles de cellulose entrant dans la composition du fluide de forage présentent environ au moins 80 % de cellules à parois primaire, et sont chargées, en surface, en acides carboxyliques, en polysaccharides acides, seuls ou en mélange.

Par acides carboxyliques, on entend les acides carboxyliques simples, ainsi que leurs sels. Ces acides sont de préférence choisis parmi les acides uroniques, ou leurs sels. Plus particulièrement, lesdits acides uroniques sont l'acide galacturonique, l'acide glucuronique, ou leurs sels.

En tant que polysaccharides acides, on peut citer les pectines, qui sont plus particulièrement des acides polygalacturoniques. Ces polysaccharides acides peuvent être présents en mélange avec des hémicelluloses.

Comme cela a été indiqué auparavant, les nanofibrilles de cellulose mises en oeuvre dans le fluide de forage sont chargées en acides et en polysaccharides. Il est à noter qu'il ne s'agit pas ici d'un simple mélange entre lesdites nanofibrilles et les acides et polysaccharides, mais plutôt d'une combinaison étroite entre ces deux types de composés. En effet, le procédé de préparation des nanofibrilles est tel que les acides et polysaccharides ne sont pas totalement séparés des fibres mais restent encore en surface de ces dernières, leur conférant des propriétés bien spécifiques. Ainsi, les nanofibrilles employées selon l'invention sont chargées en surface par des acides carboxyliques, des polysaccharides acides, seuls ou en mélange. Il faut noter qu'il n'est pas possible d'obtenir de les mêmes propriétés si ces acides et/ou polysaccharides sont totalement séparés des nanofibrilles lors de leur préparation pour leur être rajoutés par la suite.

Les nanofibrilles de cellulose entrant dans la composition du fluide selon la présente invention sont issues de cellules constituées, de préférence, d'au moins environ 80% de parois primaires. De préférence, la quantité de parois primaires est d'au moins 85 % en poids.

On a de telles caractéristiques notamment avec des cellules de parenchyme. La pulpe de betterave sucrière, les citrus comme les citrons, les oranges, les pamplemousses, et la plupart des fruits et des légumes constituent des exemples de parenchyme.

En outre, les nanofibrilles de cellulose entrant dans la composition du fluide selon l'invention sont essentiellement amorphes.

Par essentiellement amorphes, on entend des nanofibrilles dont le taux de cristallinité est inférieur ou égal à 50 %. Selon une variante particulière de la présente invention, le taux de cristallinité est compris entre 15 % et 50 %. De préférence, le taux de cristallinité est inférieur à 50 %.

Les nanofibrilles de cellulose présentent par ailleurs, une section comprise entre environ 2 et environ 10 nm. Plus particulièrement, la section des nanofibrilles est comprise entre environ 2 et environ 4 nm.

Les nanofibrilles particulières entrant dans la composition des fluides de forage selon l'invention présentent de telles caractéristiques du fait de la mise en oeuvre d'un procédé de préparation bien particulier, qui va maintenant être décrit.

Il est à noter que ce procédé a été décrit dans la demande de brevet EP 726 356, à laquelle on pourra se référer pour plus de détails.

Tout d'abord, ledit procédé est plus particulièrement effectué sur de la pulpe de végétaux à parois primaires, comme par exemple de la pulpe de betterave après que celle-ci a subi une étape d'extraction préalable du saccharose, selon les méthodes connues de la technique.

Le procédé de préparation comprend les étapes suivantes :
(a) première extraction acide ou basique, à l'issue de laquelle on récupère un premier résidu solide,
(b) éventuellement seconde extraction effectuée dans des conditions alcalines du premier résidu solide, à la suite de quoi, est récupéré un second résidu solide,
(c) lavage du premier ou du second résidu solide,
(d) éventuellement blanchiment du résidu lavé,
(e) dilution du troisième résidu solide obtenu à l'issue de l'étape (d) de manière à obtenir un taux de matières sèches compris entre 2 et 10 % en poids,
(f) homogénéisation de la suspension diluée.

Dans l'étape (a), on entend par "pulpe" de la pulpe humide, déshydratée, conservée par ensilage ou partiellement dépectinée.

L'étape d'extraction (a) peut être effectuée en milieu acide ou en milieu basique.

Pour une extraction acide, la pulpe est mise en suspension dans une solution d'eau pendant quelques minutes de façon à homogénéiser la suspension acidifiée à un pH compris entre 1 et 3, de préférence entre 1,5 et 2,5.

Cette opération est mise en oeuvre avec une solution concentrée d'un acide tel que l'acide chlorhydrique ou l'acide sulfurique.

Cette étape peut être avantageuse pour éliminer les cristaux d'oxalate de calcium qui peuvent être présents dans la pulpe, et qui, du fait de leur caractère abrasif important, peuvent causer des difficultés dans l'étape d'homogénéisation.

Pour une extraction basique, la pulpe est ajoutée à une solution alcaline d'une base, par exemple de la soude ou de la potasse, de concentration inférieure à 9 % en poids, plus particulièrement inférieure à 6 % en poids. De préférence, la concentration de la base est comprise entre 1 et 2 % en poids.

On pourra ajouter une faible quantité d'un agent antioxydant soluble dans l'eau, tel que le sulfite de sodium Na₂SO₃, afin de limiter les réactions d'oxydation de la cellulose.

L'étape (a) est effectuée en général à une température comprise entre environ 60°C et 100°C, de préférence comprise entre environ 70°C et 95°C.

La durée de l'étape (a) est comprise entre environ 1 heure et environ 4 heures.

Lors de l'étape (a), il se produit une hydrolyse partielle avec libération et solubilisation de la majeure partie des pectines et des hémicelluloses, tout en préservant la masse moléculaire de la cellulose.

Le résidu solide est récupéré à partir de la suspension provenant de l'étape (a) en mettant en oeuvre des méthodes connues. Ainsi, il est possible de séparer le résidu solide par centrifugation, par filtration sous vide ou sous pression, avec les toiles filtrantes, ou les filtres-presses par exemple, ou encore par évaporation.

On soumet éventuellement le premier résidu solide obtenu à une seconde étape d'extraction, effectuée dans des conditions alcalines.

On met en oeuvre une seconde étape d'extraction lorsque la première a été conduite dans des conditions acides. Si la première extraction a été effectuée dans des conditions alcalines, la seconde étape n'est que facultative.

Selon le procédé, cette seconde extraction est effectuée avec une base de préférence choisie parmi la soude ou la potasse, dont la concentration est inférieure à environ 9 % en poids, de préférence comprise entre environ 1 % et environ 6 % en poids.

La durée de l'étape d'extraction alcaline est comprise entre environ 1 et environ 4 heures. Elle est de préférence égale à environ 2 heures.

A l'issue de cette seconde extraction, si elle a lieu, on récupère un second résidu solide.

Dans l'étape (c) le résidu provenant de l'étape (a) ou (b) est lavé abondamment à l'eau afin de récupérer le résidu de matériau cellulosique.

Le matériau cellulosique de l'étape (c) est ensuite facultativement blanchi, dans l'étape (d), selon les méthodes classiques. Par exemple, on peut effectuer un traitement au chlorite de sodium, à l'hypochlorite de sodium, au peroxyde d'hydrogène à raison de 5-20 % par rapport à la quantité de matières sèches traitée.

Différentes concentrations d'agent de blanchiment peuvent être utilisées, à des températures comprises entre environ 18°C et 80°C, de préférence entre environ 50°C et 70°C.

La durée de cette étape (d) est comprise entre environ 1 heure et environ 4 heures, de préférence entre environ 1 et environ 2 heures.

On obtient alors un matériau cellulosique contenant entre 85 et 95 % en poids de cellulose.

A l'issue de cette étape de blanchiment, il peut être préférable de laver abondamment la cellulose avec de l'eau.

La suspension résultante, éventuellement blanchie, est ensuite rediluée dans de l'eau à raison de 2 à 10 % de matières sèches, puis subit une étape d'homogénéisation.

Celle-ci correspond à un mixage, broyage ou toute opération de cisaillement mécanique élevé, suivie d'un ou plusieurs passages de la suspension de cellules à travers un orifice de petit diamètre, soumettant la suspension à une chute de pression d'au moins 20 MPa et à une action de cisaillement à vitesse élevée suivie d'un impact de décélération à vitesse élevée.

Le mixage ou broyage est, par exemple, effectué par passage(s) au mixeur ou broyeur pendant une durée allant de quelques minutes à environ une heure, dans un appareil de type tel un WARING BLENDOR équipé d'une hélice à quatre pales ou broyeur à meule ou tout autre type de broyeur, tel un broyeur colloïdal.

L'homogénéisation proprement dite sera avantageusement effectuée dans un homogénéiseur du type MANTON GAULIN dans lequel la suspension est soumise à une action de cisaillement à vitesse et à pression élevées dans un passage étroit et contre un anneau de choc. On peut aussi citer le MICRO FLUIDIZER qui est un homogénéiseur principalement constitué d'un moteur à air comprimé qui crée de très fortes pressions, d'une chambre d'interaction dans laquelle s'effectue l'opération d'homogénéisation (cisaillement élongationnel, chocs et cavitations) et d'une chambre basse pression qui permet la dépressurisation de la dispersion.

La suspension est introduite dans l'homogénéiseur de préférence après préchauffage à une température comprise entre 40 et 120°C, de préférence comprise entre 85 et 95°C.

La température de l'opération d'homogénéisation est maintenue entre 95 et 120°C, de préférence supérieure à 100°C.

La suspension est soumise dans l'homogénéiseur à des pressions comprises entre 20 et 100 MPa, et de préférence supérieures à 50 MPa.

L'homogénéisation de la suspension cellulosique est obtenue par un nombre de passages pouvant varier entre 1 et 20, de préférence entre 2 et 5, jusqu'à obtention d'une suspension stable.

L'opération d'homogénéisation peut avantageusement être suivie d'une opération de cisaillement mécanique élevé, par exemple dans un appareil tel l'ULTRA TURRAX de SYLVERSON.

Le procédé qui vient d'être décrit permet d'obtenir des nanofibrilles qui conservent encore à leur surface des acides carboxyliques et/ou des polysaccharides, qui sont l'une des raisons de leurs propriétés bien spécifiques de résistance à la chaleur, et ceci sans l'aide d'additif viscosifiant supplémentaire.

Selon une première variante, les nanofibrilles sont utilisées sous la forme d'une suspension n'ayant pas subi de séchage après leur obtention.

Selon une seconde variante, on met en oeuvre une composition de nanofibrilles de cellulose issue du séchage d'une dispersion de nanofibrilles en présence d'un additif et éventuellement d'un co-additif. Il est de même envisageable de mettre en oeuvre dans les fluides de forage, une dispersion d'une telle composition.

De telles compositions ont notamment fait l'objet des demandes de brevets suivantes : FR 9609061 du 15/07/96, FR 9611986 du 27/09/96, FR 9609062 du 15/07/96 et FR 9611779 du 27/09/96 ; brevets auxquels on pourra se référer pour ce qui concerne la nature des additifs, co-additifs et combinaisons préférées, les proportions respectives des additifs et co-additifs, ainsi que leur mode de préparation.

Selon une première possibilité, la composition de nanofibrilles comprend de la cellulose carboxylée, de préférence carboxyméthylée, présentant un degré de substitution inférieur ou égal à 0,95 en tant qu'additif.

Ces compositions peuvent éventuellement comprendre au moins un co-additif choisi parmi :
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères, ces co-additifs pouvant être utilisés seuls ou en mélange.

Une seconde possibilité consiste à mettre en oeuvre des compositions comprenant les nanofibrilles avec, en tant qu'additif, de la cellulose carboxylée, sous forme sel ou acide, présentant un degré de substitution supérieur à 0,95, un polysaccharide naturel, un polyol ; ces additifs pouvant être employés seuls ou en combinaison.

Le polysaccharide naturel peut être d'origine bactérienne, animale ou végétale.

Les polysaccharides sont des polymères comprenant des unités osidiques. De préférence, on met en oeuvre des polysaccharides se trouvant sous une forme anionique ou non ionique.

Parmi les polysaccharides anioniques convenables, on peut mentionner sans intention de s'y limiter, la gomme xanthane, les succinoglycanes, les carraghénanes, les alginates.

A titre de polysaccharides non ioniques, on peut citer par exemple les galactomannanes comme la gomme de guar, la gomme de caroube, l'amidon et ses dérivés non ioniques, ainsi que les dérivés non ioniques de la cellulose.

Parmi les polyols convenables, on peut citer tout particulièrement l'alcool polyvinylique.

Lesdites composition peuvent en outre comprendre, éventuellement au moins un co-additif choisi parmi l'un ou plusieurs des composés suivants :
- la cellulose carboxylée présentant un degré de substitution inférieur ou égal à 0,95, de préférence la cellulose carboxyméthylée,
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères,

Dans l'une et l'autre des possibilités, la teneur en additif et éventuellement en co-additif, est inférieure à 30 % en poids par rapport au poids de nanofibrilles et d'additif et de co-additif.

Le procédé de préparation de telles compositions de nanofibrilles consiste, tout d'abord, à préparer les nanofibrilles de cellulose à partir de pulpe cellulosique appropriée en effectuant une hydrolyse puis éventuellement au moins une étape de blanchiment de la pulpe ainsi traitée. Ce qui a été indiqué auparavant à ce propos reste valable et ne sera pas repris ici.

Plus particulièrement, dans une première étape, on ajoute à la suspension de nanofibrilles, éventuellement ayant subi au moins un cycle d'homogénéisation, au moins une partie de l'additif et éventuellement du ou des co-additifs. Puis, dans une seconde étape, on met en oeuvre une étape de séchage de la suspension ainsi additivée.

Selon une première variante, l'addition d'au moins une partie de l'additif et éventuellement du co-additif, est effectuée à l'issue de l'étape d'homogénéisation.

Une première méthode particulièrement appropriée, consiste à ajouter au moins une partie de l'additif et éventuellement du co-additif, à la suspension à l'issue de l'étape d'homogénéisation, après que cette dernière a subi au moins une étape de concentration.

A titre indicatif, la ou les étapes de concentration ont lieu par filtration, centrifugation, ou encore évaporation d'une partie de l'eau de la suspension, par précipitation, par exemple dans un alcool, par congélation-décongélation, par dialyse, etc.

Selon ce mode de réalisation, l'opération de concentration peut être conduite jusqu'à obtenir un extrait sec d'environ 35 % en poids.

L'introduction de l'additif et éventuellement du co-additif est effectuée de manière connue en soi, c'est-à-dire par tout moyen permettant d'introduire de manière homogène une solution, une suspension ou une poudre, à une suspension qui a plutôt la consistance d'une pâte. Par exemple, on peut citer les broyeurs, les extrudeurs, les malaxeurs.

Cette opération peut être effectuée dans une large gamme de température, comprise plus particulièrement entre la température ambiante et 80°C.

Une seconde méthode à ajouter au moins une partie de l'additif et éventuellement du co-additif, à la suspension à l'issue de l'étape d'homogénéisation, avant que cette dernière ait subi au moins une étape de concentration.

Dans ce cas, l'étape ou les étapes de concentration qui ont lieu après l'ajout d'additif et éventuellement du co-additif, sont effectuées de la même manière que précédemment indiqué.

Une troisième méthode de mise en oeuvre de la première variante, consiste à introduire l'additif après que la suspension a subi une ou plusieurs étapes de concentration.

Selon une deuxième variante avantageuse, l'introduction d'au moins une partie de l'additif et éventuellement du co-additif est effectuée avant ou pendant l'étape d'homogénéisation. Lorsqu'il est indiqué que l'additivation a lieu pendant l'étape d'homogénéisation, on entend que l'additif et éventuellement le co-additif sont introduits alors que la pulpe a subi au moins un cycle de l'étape d'homogénéisation.

L'additivation a lieu selon les trois méthodes indiquées dans le cadre de la première variante.

Préalablement à l'étape de séchage proprement dite, il peut être avantageux d'effectuer une mise en forme de la suspension qui a été concentrée comme mentionné auparavant. Cette mise en forme est réalisée de manière connue de l'homme du métier. On peut notamment citer, sans intention de s'y limiter toutefois, l'extrusion, la granulation.

Selon une variante particulièrement avantageuse, on effectue l'étape de séchage de manière à maintenir au minimum 3 % en poids d'eau par rapport au poids du solide obtenu. Plus particulièrement, le poids d'eau maintenu est compris entre 10 et 30% en poids.

Le séchage a lieu de manière avantageuse sous air, bien qu'il soit envisageable de le mettre en oeuvre sous un gaz inerte, comme l'azote.

Il est en outre à noter que l'on préfère mettre en oeuvre le séchage sous une atmosphère dont le degré d'humidité est contrôlé de manière à pouvoir maintenir le taux d'humidité souhaité dans la composition.

La température de séchage doit limiter toute dégradation des acides carboxyliques, des polysaccharides acides, des hémicelluloses et/ou des additifs et co-additifs. Elle est plus particulièrement comprise entre 30 et 80°C, de préférence entre 30 et 60°C.

Il est à noter que l'on ne sortirait pas du cadre de la présente invention en mettant en oeuvre un séchage en plusieurs étapes, dont certaines d'entre elles mettraient en oeuvre les moyens indiqués précédemment pour l'étape de concentration.

A l'issue de l'étape de séchage, on peut effectuer un broyage de la composition obtenue.

La composition comprenant les nanofibrilles, au moins un additif et éventuellement au moins un co-additif, peuvent être utilisées pour la préparation de fluide de forage selon l'invention, sous cette forme, mais aussi sous la forme d'une suspension de nanofibrilles de cellulose obtenue par redispersion dans l'eau ou tout autre milieu, de la composition précitée.

La teneur en nanofibrilles de cellulose dans le fluide de forage peut varier dans de larges limites. Cependant, elle est avantageusement comprise entre 0,05 et 2% par rapport au poids total du fluide, et de préférence comprise entre 0,05 et 1 %.

Il est à noter que l'effet viscosant de l'additif selon l'invention est plus marqué, à teneur comparable, à celle des polysaccharides mis en oeuvre habituellement. Ainsi, il est possible de diminuer la quantité de l'additif sans effet néfaste sur les propriétés rhéologiques du fluide de forage.

Le fluide selon l'invention peut comprendre, outre les nanofibrilles de cellulose, un composé réducteur de filtrat. On entend par réducteur de filtrat des composés s'adsorbant sur les roches constituant les parois du puits, limitant de ce fait la diffusion à travers les parois du forage des divers éléments constitutifs du fluide.

A titre d'exemple de composés de ce type, on peut citer sans intention de s'y limiter, les composés cellulosiques, les polyacrylamides, les polyacrylates de haut poids moléculaire, les succinoglycanes, l'amidon natif ou ses dérivés, le charbon. Parmi les composés cellulosiques, les celluloses non modifiées ou modifiées chimiquement comme les carboxyméthylcelluloses, les hydroxyéthylcelluloses, les carboxyéthyl-hydroxyéthylcelluloses sont des composés convenant comme réducteur de filtrat. Bien entendu rien n'empêche d'employer ces produits en combinaison si les conditions le rendent nécessaire.

La quantité de réducteur de filtrat dépend fortement de la nature des roches traversées. Cependant, à titre indicatif, celle-ci est habituellement comprise entre 0 et 1% par rapport au poids total du fluide.

Les fluides de forages peuvent comprendre aussi des agents fluidifiants ou dispersants. Ainsi, peuvent entrer dans la composition des fluides de forages, des polyphosphates, des tannins, des lignosulfonates, des dérivés de lignine, des tourbes et lignites, des polyacrylates, des polynaphtalène sulfonates, seuls ou en mélange.

La quantité d'agent fluidifiant ou dispersant est variable. A titre indicatif, celle-ci est comprise entre 0 et 1% par rapport au poids total du fluide.

Le fluide de forage selon l'invention peut comprendre en outre un capteur d'oxygène. Ce type d'additif a pour objet de piéger l'oxygène présent dans les boues de forages et qui peuvent entraîner une dégradation de certains additifs.

Parmi les produits de ce type, on peut citer par exemple les hydroxylamines, l'hydrazine, les sulfites, les bisulfites, les hydrosulfites, les borohydrures.

Selon un mode de réalisation particulier, on utilise l'hydrazine comme capteur d'oxygène car elle n'entraîne pas la formation de précipités insolubles favorisant l'apparition de bouchons dans le puits. L'hydrazine peut se trouver sous une forme anhydre ou hydratée, sous forme de sels comme par exemples les chlorure, sulfate, ou encore sous forme de carbohydrazide.

Généralement la teneur en additif de ce type varie entre 0 et 0,25%.

Le fluide de forage selon l'invention peut comprendre de plus, au moins un composé alourdissant et/ou au moins un colloïde minéral.

Les éléments alourdissants contribuent à maintenir une pression hydrostatique suffisante dans le puits et à maintenir en suspension les roches entraînées lors de l'opération de forage. De tels composés sont classiquement choisis parmi les sulfates, silicates ou carbonates de métaux alcalino-terreux, comme le sulfate de baryum, le carbonate de calcium, les silicates de potassium et de sodium. On peut de même utiliser des bromures de métaux alcalino-terreux ou de zinc tels que le bromure de potassium, le bromure de zinc. On peut aussi utiliser des oxydes fer.

Les colloïdes minéraux, qui sont des composés substantiellement insolubles dans les conditions d'utilisation du fluide selon l'invention, sont des agents modifiant la rhéologie du milieu et permettant de maintenir les déblais en suspension dans ce dernier. L'attapulgite, la baryte, la bentonite, seules ou en mélange, en sont les exemples les plus couramment utilisés. Il est à noter que la bentonite, en présence d'un milieu aqueux non salin, joue aussi un rôle de réducteur de filtrat.

Bien que les colloïdes minéraux ne sont pas des éléments toujours requis dans la composition des fluides de forage, un fluide particulièrement avantageux comprend une association des nanofibrilles de cellulose qui ont été décrites précédemment, avec au moins un colloïde minéral. De préférence, on utilise la bentonite.

Il est à noter que si le milieu aqueux est salin, on utilise de préférence de l'attapulgite comme colloïde minéral, en association avec les nanofibrilles.

Les teneurs en alourdissants et en colloïdes minéraux dépendent de plusieurs facteurs qui ne sont pas uniquement techniques. En effet, si ces teneurs sont bien évidemment fixées en fonction de la nature des sols traversés, l'importance du coût engendré par l'usage de ces additifs est prise en compte (présence sur place ou non, coût, etc.).

Bien souvent, et toujours dans le but de minimiser les frais encourus, la préparation du fluide de forage est réalisée avec l'eau présente sur le site de forage.

Ainsi, il n'est pas rare de se trouver en présence d'eau de formation (par opposition au eaux de composition, c'est-à-dire aux eaux préparées dans un but particulier) chargées en sels, comme l'eau de mer, les eaux saumurées ou les eaux dures. Dans ce cas, la teneur en sels dans l'eau employée varie selon la provenance de celle-ci.

Il peut toutefois arriver que l'eau disponible soit de l'eau non ou peu chargée.
Dans ce cas, il peut être approprié d'ajouter des sels, tels que des chlorures par exemple. En effet, si la roche traversée a tendance à gonfler en présence d'eau, et entraîner des problèmes de bouchage lors du forage, la présence de sels de ce type contribue à diminuer cet inconvénient.

Les sels employés habituellement dans cette optique sont des halogénures, et notamment des iodures ou des chlorures, des sulfates, des carbonates, des bicarbonates, des phosphates, des silicates de métaux alcalins, seuls ou en mélange. Ainsi, les sels de sodium, de potassium, font partie des sels les plus couramment employés.

On peut également ajouter, si nécessaire, des sels minéraux pour favoriser la précipitation de certains ions, s'ils sont présents, et en particulier des ions divalents. On peut mentionner par exemple l'addition de carbonate de soude pour précipiter le calcium, ou le bicarbonate de soude pour précipiter la chaux, notamment lors de reforages dans le ciment. On peut encore citer l'addition de gypse ou de chlorure de calcium pour limiter le gonflement des argiles, l'addition d'hydroxyde de calcium, ou de chaux éteinte, pour débicarbonater des boues contaminées par du dioxyde de carbone.

La teneur en sels est là encore fonction des roches traversées et des eaux disponibles sur le site d'exploitation et l'on peut effectuer les opérations en présence de fluides saturés en sels.

On peut aussi mettre en oeuvre des fluides pour lesquels la teneur en sels de ce type est comprise entre 0,01 et 2 % en poids par rapport au fluide de forage.

Un fluide de forage particulièrement intéressant selon l'invention comprend, outre les nanofibrilles qui ont été décrites auparavant, au moins un sel minéral, chois parmi ceux qui ont été mentionnés auparavant, ou leurs combinaisons.

Bien évidemment, le fluide de forage selon la présente invention peut comprendre des additifs habituels de la classe des polysaccharides de haut poids moléculaire, comme la gomme xanthane ou encore le guar, utile en tant que viscosants.

D'autres additifs classiques pour des applications concernant l'exploitation de gisements pétroliers peuvent entrer dans la composition du fluide. Ainsi, on peut mentionner les agents de transfert de radicaux libres, comme les alcools inférieurs, les thiourées, l'hydroquinone ; les biocides, les agents chélatants, les tensioactifs, des anti-mousses, des agents anticorrosion par exemple.

Ainsi que cela a été indiqué dans ce qui a précédé, le fluide selon la présente invention est particulièrement approprié pour être utilisé en tant que fluide de forage.

A ce sujet, tous les types de forage peuvent convenir, qu'ils soient verticaux, horizontaux ou obliques, tels que ceux qui sont pratiqués sur les plates-formes off shore.

Il est à noter que grâce à ses propriétés (compatibilité avec de nombreux composés notamment) le fluide selon l'invention ne contamine pas le ciment lors des opérations de "work-over". Ces opérations consistent, une fois le forage du puits terminé, à introduire un caisson métallique dans ce puits pour le consolider, puis à couler un ciment entre ce caisson et la paroi du puits.

En outre, moyennant une adaptation des caractéristiques dudit fluide (rhéologie, composition), on peut employer le fluide selon l'invention comme fluide espaceur, par exemple.

Le fluide selon l'invention, toujours après adaptation de sa composition et de ses propriétés rhéologiques, peut de même être mis en oeuvre lors de l'exploitation même du gisement pétrolier, notamment dans la récupération assistée du pétrole. Ainsi, on peut l'employer comme fluide de stimulation, qui représente l'une des méthodes développées pour augmenter le rendement de l'exploitation d'un gisement de pétrole.
En effet, ce fluide est introduit dans un autre endroit du gisement et du fait de sa viscosité importante permet l'entraînement d'une quantité supplémentaire de pétrole augmentant ainsi le rendement de l'extraction.

Des exemples concrets mais non limitatifs de l'invention vont maintenant être présentés.

### EXEMPLE 1

### 1/ Préparation des boues

On prépare une boue de composition suivante :

| | |
|---|---|
| suspension aqueuse de bentonite (5 %) | 157,50 g |
| eau de mer | 140,30 g |
| réducteur de filtrat : amidon modifié DRISPAC (Drilling Specialties Company) | 0,50 g |
| NaHCO₃ | 0,65 g |
| Na₂CO₃ | 0,22 g |
| dispersant : polyacrylate colloid 211 (Rhône-Poulenc) | 0,75 g |
| baryte | 197,20 g |
| additif rhéologique en suspension | 66,6 g |

La formulation est préparée de telle sorte que la quantité d'additif rhéologique est de 0,28 % en poids par rapport à la phase aqueuse.

### Exemple a) selon l'invention :

L'additif rhéologique est constitué de 66,6 g d'une suspension de nanofibrilles de cellulose, obtenues selon l'exemple 20 de la demande de brevet européen EP°726 356, au nom de Générale Sucrière.

### Exemple b) comparatif :

L'additif rhéologique est constitué par une solution de 66,6 g de gomme xanthane (RHODOPOL 23P® , Rhône-Poulenc). L'extrait sec de la solution est de 1,5 %.

On prépare tout d'abord la suspension aqueuse de bentonite par hydratation pendant au moins 16 heures d'une suspension comprenant 5% en poids de bentonite dans de l'eau de ville.

La préparation des boues a) et b) est effectuée dans un mélangeur Hamilton Beach.

On mélange donc la suspension de bentonite avec l'eau de mer, puis on ajoute l'additif rhéologique en solution. On mélange l'ensemble pendant 5 minutes, puis on ajoute le bicarbonate de sodium, puis le carbonate de sodium, le dispersant. On mélange l'ensemble pendant 3 minutes. On ajoute ensuite le réducteur de filtrat et enfin la baryte et l'on continue l'opération d'homogénéisation jusqu'à une durée totale de mélange de 25 minutes.

On obtient alors deux boues a) et b) présentant une densité de 1,4.

### 2/ Comportement rhéologique des boues

On procède à des mesures du comportement rhéologique des boues après leur fabrication et après un traitement à 120°C dans une étuve de roulage pendant 24 heures.

Dans le cas de la boue selon l'invention, on effectue aussi ces mesures sur la boue ayant subi un traitement semblable à celui indiqué précédemment mais à 140°C.

Les mesures sont effectuées avec un rhéomètre Fann (BAROID) selon les caractéristiques indiquées dans American Petroleum Institute (API) dans le bulletin 13 D.

Ces mesures sont effectuées à la température de 21 ± 1°C, par lecture directe de la déviation du fil de tension. Les vitesses de rotation sont 600, 300, 200 et 100 tr/min.

L'exploitation de ces résultats permet de définir la viscosité apparente des boues (Va exprimée en mPa.s), la viscosité plastique (Vp exprimée en mPa.s) et le "Yield point" (Yp exprimé en Pa).

Elle permet aussi d'accéder aux profils rhéologiques d'écoulement des boues par correspondance d'une part entre les tr/min du rhéomètre et les gradients en s-1 et les déviations du fil de torsion et les viscosités apparentes.

### Les résultats sont rassemblés dans les tableaux 1 et 2 suivants :

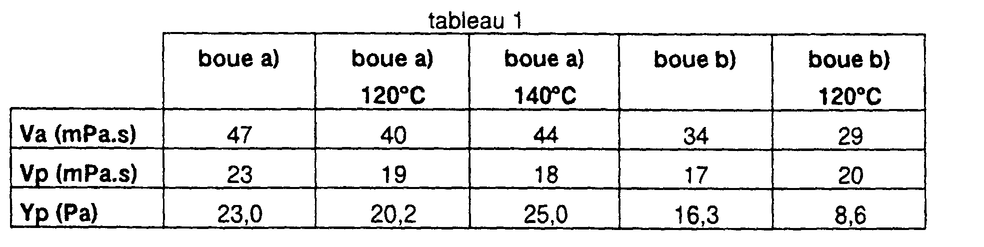

On constate d'après ces résultats qu'à isoconcentration en additif rhéologique, les valeurs de viscosités apparentes sont plus élevées dans le cas de l'additif selon l'invention. Ceci implique donc que l'additif selon l'invention présente un pouvoir viscosifiant supérieur à celui de la gomme xanthane.

Par ailleurs, les valeurs plus élevées en Yp obtenues avec l'additif selon l'invention montrent que ce dernier présente des propriétés suspensives plus importantes que celles de la gomme xanthane.

On constate enfin que l'essentiel des propriétés rhéologiques de la boue comprenant l'additif selon l'invention sont maintenues après un traitement allant jusqu'à 140°C, contrairement à la boue additivée de manière classique.

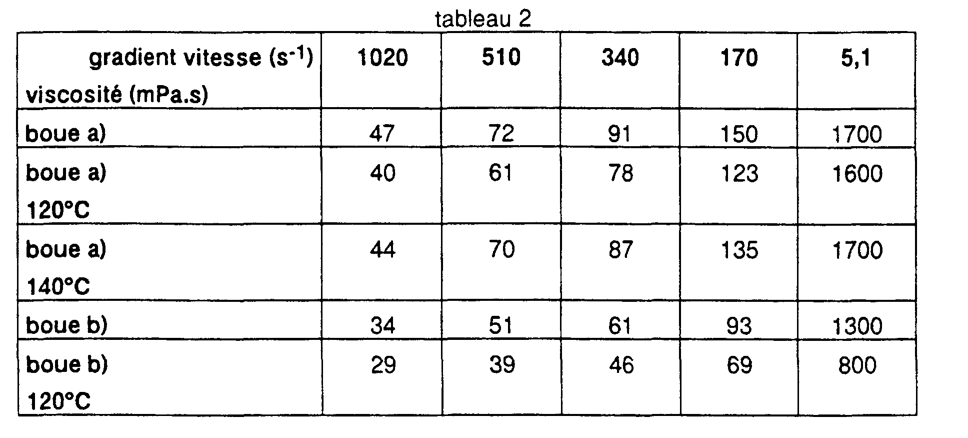

Le calcul de la pente du diagramme logarithme de la viscosité en fonction du logarithme du gradient de vitesse, montre que l'on obtient pour chacune des boues une pente négative, inférieure à -0,5, ce qui est caractéristique de boues de forage adéquates pour l'application, c'est-à-dire qu'elle présentent de bonnes propriétés de conservation des déblais en suspension, évitant de ce fait la dépôt de ces derniers dans le fond du puits, occasionnant son bouchage.

On vérifie aussi qu'à isoconcentration et avec le même gradient de vitesse, les propriétés viscosifiantes de la boues selon l'invention sont supérieures à celles de la boue comprenant la gomme xanthane en tant qu'additif rhéologique.

### EXEMPLE 2

Cet essai illustre la stabilité des nanofibrilles de cellulose, utilisées comme seul viscosifiant vis-à-vis de la température.

On prépare une suspension aqueuse de nanofibrilles de cellulose, obtenues selon l'exemple 20 de la demande de brevet européen EP 96 400 261.2 précitée, à une concentration de 1,68 % (soit 6 ppb).

La mise en suspension est réalisée conformément à la norme API Section 13 B, au moyen d'un appareillage Hamilton Beach, pendant 30 minutes à vitesse lente.

Le comportement rhéologique de la solution est contrôlée après la fabrication et après un traitement thermique à 180°C en étuve, la suspension étant placée dans une cellule en inox pressurisée par de l'azote, puis roulée pendant 16 heures.

Les viscosités (exprimées en mPa.s) sont rassemblées dans le tableau suivant :

**tableau 3**

| **Gradient (s**^{**-1**}**)** | **1020** | **510** | **340** | **170** | **10,2** | **5,1** |
|---|---|---|---|---|---|---|
| **avant traitement** | 87 | 130 | 171 | 270 | 1200 | 1800 |
| **après traitement** | 59 | 95 | 122 | 183 | 900 | 1400 |
| NB : Les valeurs rassemblées dans le tableau ci-dessus seront à adapter par l'homme de métier en fonction des applications du fluide. Par exemple, pour des applications comme fluide de forage, on emploiera de préférence une teneur en additif inférieure à 1 %. | | | | | | |

On constate que dans des conditions extrêmes, l'additif conserve l'essentiel de ses propriétés et son caractère pseudo-plastique après un traitement thermique très dur. Par conséquent, lors de l'utilisation de cet additif dans les boues de forage, la quantité pourra rester de manière avantageuse, limitée.

## Revendications

1. Fluide de forage mis en oeuvre en présence d'eau, comprenant une composition de nanofibrilles de cellulose issues de cellules constituées d'au moins 80 % de parois primaires et possédant un taux de cristallinité inférieur ou égal à 50 %, et chargées, en surface, en acides carboxyliques, en polysaccharides acides, seuls ou en mélange, ladite composition étant issue du séchage d'une dispersion de nanofibrilles en présence d'un additif, et éventuellement d'un co-additif.

2. Fluide de forage mis en oeuvre en présence d'eau, comprenant une dispersion d'une composition selon la revendication 1.

3. Fluide de forage selon l'une quelconque des revendications précédentes, **caractérisé en ce que** les acides carboxyliques sont des acides uroniques ou leurs sels, les polysaccharides sont des pectines, seules ou en mélange avec des hémicelluloses.

4. Fluide de forage selon l'une quelconque des revendications 1 à 3, **caractérisé en ce que** les nanofibrilles possèdent un taux de cristallinité inférieur à 50 %.

5. Fluide de forage selon l'une quelconque des revendications 1 à 4, **caractérisé en ce que** les nanofibrilles présentent une section comprise entre 2 et environ 10 nm, plus particulièrement entre 2 et environ 4 nm.

6. Fluide de forage selon l'une quelconque des revendications 1 à 5, **caractérisé en ce que** l'additif est de la cellulose carboxylée, de préférence carboxyméthylée, avec un degré de substitution inférieur ou égal à 0,95.

7. Fluide de forage selon l'une quelconque des revendications 1 à 5, **caractérisé en ce que** l'additif est de la cellulose carboxylée, sous forme sel ou acide, avec un degré de substitution supérieur à 0,95, un polysaccharide naturel, un polyol, seuls ou en combinaison.

8. Fluide de forage selon l'une quelconque des revendications 1 à 6, **caractérisé en ce que** le co-additif est choisi parmi :
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères, seuls ou en mélange.

9. Fluide de forage selon l'une quelconque des revendications 1 à 5 et 7, **caractérisé en ce que** le co-additif est choisi parmi l'un ou plusieurs des composés suivants :
- la cellulose carboxylée présentant un degré de substitution inférieur ou égal à 0,95, de préférence la cellulose carboxyméthylée,
- les monomères ou oligomères osidiques,
- les composés de formule (R¹R²N)COA, formule dans laquelle R¹ ou R², identiques ou différents, représentent l'hydrogène ou un radical alkyle en C₁-C₁₀, de préférence en C₁-C₅, A représente l'hydrogène, un radical alkyle en C₁-C₁₀, de préférence en C₁-C₅, ou encore le groupement R'¹R'²N avec R'¹, R'², identiques ou différents, représentant l'hydrogène ou un radical alkyle en C₁-C₁₀, de préférence en C₁-C₅,
- les tensioactifs cationiques ou amphotères.

10. Fluide de forage selon l'une quelconque des revendications 1 à 9, **caractérisé en ce que** la teneur en additif et éventuellement en co-additif est inférieure à 30 % en poids par rapport au poids de nanofibrilles et d'additif et de co-additif.

11. Fluide de forage selon l'une quelconque des revendications précédentes, **caractérisé en ce que** la teneur en nanofibrilles de cellulose est comprise entre 0,05 et 2 % par rapport au poids total de fluide, de préférence 0,05 à 1 %.

12. Fluide de forage selon l'une quelconque des revendications précédentes, **caractérisé en ce que** la composition est issue d'un séchage dont la température doit limiter toute dégradation des acides carboxyliques, des polysaccharides acides, des hémicelluloses et/ou des additifs et co-additifs.

13. Fluide de forage selon la revendication 12, **caractérisé en ce que** la température est comprise entre 30 et 80°C, de préférence entre 30 et 60°C.

14. Fluide de forage selon l'une quelconque des revendications précédentes, **caractérisé en ce qu'**il comprend un réducteur de filtrat, avec une quantité comprise entre 0 et 1 % par rapport au poids total du fluide.

15. Fluide de forage selon la revendication précédente, **caractérisé en ce que** le réducteur de filtrat est choisi parmi les composés cellulosiques, les polyacrylamides, les polyacrylates de haut poids moléculaire, les succinoglycanes, l'amidon natif ou ses dérivés, le charbon, seuls ou en combinaison.

16. Fluide de forage selon l'une quelconque des revendications précédentes, **caractérisé en ce qu'**il comprend un agent fluidifiant ou dispersant avec une quantité comprise entre 0 et 1 % par rapport au poids total du fluide.

17. Fluide de forage selon la revendication précédente, **caractérisé en ce que** l'agent fluidifiant ou dispersant est choisi parmi les polyphosphates, les tanins, les lignosulfonates, les dérivés de lignine, les tourbes, les lignites, les polyacrylates, les polynaphtalènes sulfonates, seuls ou en mélange.

18. Fluide de forage selon l'une quelconque des revendications précédentes, **caractérisé en ce qu'**il comprend un capteur d'oxygène avec une teneur comprise entre 0 et 0,25 % par rapport au poids total du fluide.

19. Fluide de forage selon l'une des revendications précédentes, **caractérisé en ce qu'**il comprend un composé alourdissant choisi parmi les sulfates, les carbonates, les silicates de métaux alcalino-terreux, les bromures de métaux alcalino-terreux ou de zinc, les oxydes de fer.

20. Fluide de forage selon l'une quelconque des revendications précédentes, **caractérisé en ce qu'**il comprend au moins un colloïde minéral, choisi parmi l'attapulgite, la baryte, la bentonite, seules ou en mélange.

21. Fluide de forage selon l'une quelconque des revendications précédentes, **caractérisé en ce que** qu'il comprend des sels.

22. Fluide de forage selon la revendication précédente, **caractérisé en ce que** les sels sont choisis parmi les halogénures, les sulfates, les carbonates, les bicarbonates, les silicates, les phosphates de métaux alcalins, seuls ou en mélange.

23. Fluide de forage selon l'une des revendications 21 ou 22, **caractérisé en ce que** la teneur en sels est comprise entre 0,01 et 2 % par rapport au poids total du fluide.

## Patentansprüche

1. Bohrflüssigkeit, die in Gegenwart von Wasser eingesetzt wird, umfassend eine Zusammensetzung von Cellulose-Nanofibrillen, die aus Zellen, die zu mindestens 80% aus Primärwänden gebildet werden, stammen und einen Kristallinitätsgrad unter oder gleich 50% aufweisen und an der Oberfläche mit Carbonsäuren, mit sauren Polysacchariden, allein oder in einer Mischung, beladen sind, wobei die Zusammensetzung aus dem Trocknen einer Dispersion von Nanofibrillen in Gegenwart eines Additivs und gegebenenfalls eines Coadditivs hervorgegangen ist.

2. Bohrflüssigkeit, die in Gegenwart von Wasser eingesetzt wird, die eine Dispersion einer Zusammensetzung nach Anspruch 1 umfasst.

3. Bohrflüssigkeit nach einem der vorangegangenen Ansprüche,
**dadurch gekennzeichnet, dass** die Carbonsäuren Uronsäuren oder deren Salze sind, die Polysaccharide Pectine, allein oder in einer Mischung mit Hemicellulosen, sind.

4. Bohrflüssigkeit nach einem der Ansprüche 1 bis 3, **dadurch gekennzeichnet, dass** die Nanofibrillen einen Kristallinitätsgrad unter 50% aufweisen.

5. Bohrflüssigkeit nach einem der Ansprüche 1 bis 4, **dadurch gekennzeichnet, dass** die Nanofibrillen einen Querschnitt zwischen 2 und ungefähr 10 nm, insbesondere zwischen 2 und ungefähr 4 nm aufweisen.

6. Bohrflüssigkeit nach einem der Ansprüche 1 bis 5, **dadurch gekennzeichnet, dass** das Additiv carboxylierte Cellulose, vorzugsweise carboxymethylierte Cellulose, mit einem Substitutionsgrad unter oder gleich 0,95 ist.

7. Bohrflüssigkeit nach einem der Ansprüche 1 bis 5, **dadurch gekennzeichnet, dass** das Additiv carboxylierte Cellulose, in Form eines Salzes oder einer Säure, mit einem Substitutionsgrad über 0,95, ein natürliches Polysaccharid, ein Polyol, allein oder in Kombination, ist.

8. Bohrflüssigkeit nach einem der Ansprüche 1 bis 6, **dadurch gekennzeichnet, dass** das Coadditiv ausgewählt ist unter:
- den Osidmonomeren oder -oligomeren,
- den Verbindungen der Formel (R¹R²N)COA, in welcher Formel R¹ oder R², die gleich oder verschieden sind, für Wasserstoff oder einen C₁-C₁₀-Alkylrest, vorzugsweise C₁-C₅-Alkylrest stehen, A für Wasserstoff, einen C₁-C₁₀-Alkylrest, vorzugsweise C₁-C₅-Alkylrest, oder ferner die Gruppe R'¹R'²N, wobei R'¹, R'², die gleich oder verschieden sind, für Wasserstoff oder einen C₁-C₁₀-Alkylrest, vorzugsweise C₁-C₅-Alkylrest stehen, steht,
- den kationischen oder amphoteren grenzflächenaktiven Mitteln,
allein oder in einer Mischung.

9. Bohrflüssigkeit nach einem der Ansprüche 1 bis 5 und 7,
**dadurch gekennzeichnet, dass** das Coadditiv unter einer oder mehreren der folgenden Verbindungen ausgewählt ist:
- carboxylierter Cellulose, die einen Substitutionsgrad unter oder gleich 0,95 aufweist, vorzugsweise carboxymethylierter Cellulose,
- den Osidmonomeren oder -oligomeren,
- den Verbindungen der Formel (R¹R²N)COA, in welcher Formel R¹ oder R², die gleich oder verschieden sind, für Wasserstoff oder einen C₁-C₁₀-Alkylrest, vorzugsweise C₁-C₅-Alkylrest stehen, A für Wasserstoff, einen C₁-C₁₀-Alkylrest, vorzugsweise C₁-C₅-Alkylrest, oder ferner die Gruppe R'¹R'²N, wobei R'¹, R'², die gleich oder verschieden sind, für Wasserstoff oder einen C₁-C₁₀-Alkylrest, vorzugsweise C₁-C₅-Alkylrest, stehen, steht, den kationischen oder amphoteren grenzflächenaktiven Mitteln.

10. Bohrflüssigkeit nach einem der Ansprüche 1 bis 9, **dadurch gekennzeichnet, dass** der Gehalt an Additiv und gegebenenfalls an Coadditiv unter 30 Gew.-% bezogen auf das Gewicht von Nanofibrillen und von Additiv und von Coadditiv liegt.

11. Bohrflüssigkeit nach einem der vorangegangenen Ansprüche,
**dadurch gekennzeichnet, dass** der Gehalt an Cellulose-Nanofibrillen zwischen 0,05 und 2% bezogen auf das Gesamtgewicht der Flüssigkeit, vorzugsweise zwischen 0,05 und 1% beträgt.

12. Bohrflüssigkeit nach einem der vorangegangenen Ansprüche,
**dadurch gekennzeichnet, dass** die Zusammensetzung aus einer Trocknung stammt, deren Temperatur einen jeglichen Abbau der Carbonsäure, der sauren Polysaccharide, der Hemicellulose und/oder der Additive und Coadditive zu begrenzen hat.

13. Bohrflüssigkeit nach Anspruch 12, **dadurch gekennzeichnet,**
**dass** die Temperatur zwischen 30 und 80°C, vorzugsweise zwischen 30 und 60°C liegt.

14. Bohrflüssigkeit nach einem der vorangegangenen Ansprüche,
**dadurch gekennzeichnet, dass** sie einen Filtratverminderer mit einer Menge zwischen 0 und 1% bezogen auf das Gesamtgewicht der Flüssigkeit umfasst.

15. Bohrflüssigkeit nach dem vorangegangenen Anspruch, **dadurch gekennzeichnet, dass** der Filtratverminderer unter den cellulosischen Verbindungen, den Polyacrylamiden, den Polyacrylaten mit hohem Molekulargewicht, den Succinoglykanen, nativer Stärke oder deren Derivaten, Kohle, allein oder in Kombination, ausgewählt ist.

16. Bohrflüssigkeit nach einem der vorangegangenen Ansprüche,
**dadurch gekennzeichnet, dass** sie einen Verflüssiger oder ein Dispergiermittel mit einer Menge zwischen 0 und 1% bezogen auf das Gesamtgewicht der Flüssigkeit umfasst.

17. Bohrflüssigkeit nach dem vorangegangenen Anspruch, **dadurch gekennzeichnet, dass** der Verflüssiger oder das Dispergiermittel unter den Polyphosphaten, den Tanninen, den Lignosulfaten, den Ligninderivaten, den Torfen, den Ligniten, den Polyacrylaten, den sulfonierten Polynaphthalinen, allein oder in einer Mischung, ausgewählt ist.

18. Bohrflüssigkeit nach einem der vorangegangenen Ansprüche,
**dadurch gekennzeichnet, dass** sie einen Sauerstofffänger mit einem Gehalt zwischen 0 und 0,25% bezogen auf das Gesamtgewicht der Flüssigkeit umfasst.

19. Bohrflüssigkeit nach einem der vorangegangenen Ansprüche,
**dadurch gekennzeichnet, dass** sie eine Beschwerungsverbindung, ausgewählt unter den Sulfaten, Carbonaten, Silicaten von Erdalkalimetallen, den Bromiden von Erdalkalimetallen oder von Zink, den Eisenoxiden, umfasst.

20. Bohrflüssigkeit nach einem der vorangegangenen Ansprüche, **dadurch gekennzeichnet, dass** sie mindestens ein kolloides Mineral, ausgewählt unter Attapulgit, Baryt, Bentonit, allein oder in einer Mischung, umfasst.

21. Bohrflüssigkeit nach einem der vorangegangenen Ansprüche,
**dadurch gekennzeichnet, dass** sie Salze umfasst.

22. Bohrflüssigkeit nach dem vorangegangenen Anspruch, **dadurch gekennzeichnet, dass** die Salze unter den Halogeniden, Sulfaten, Carbonaten, Bicarbonaten, Silicaten, Phosphaten von Alkalimetallen, allein oder in einer Mischung, ausgewählt sind.

23. Bohrflüssigkeit nach einem der Ansprüche 21 oder 22, **dadurch gekennzeichnet, dass** der Gehalt an Salzen zwischen 0,01 und 2% bezogen auf das Gesamtgewicht der Flüssigkeit beträgt.

## Claims

1. Drilling fluid used in the presence of water, comprising a composition of cellulose nanofibrils obtained from cells consisting of at least 80% primary walls and having a degree of crystallinity of less than or equal to 50%, and charged, at the surface, with carboxylic acids and acidic polysaccharides, alone or as a mixture, the said composition being obtained from the drying of a dispersion of nanofibrils in the presence of an additive, and optionally of a co-additive.

2. Drilling fluid used in the presence of water, comprising a dispersion of a composition according to claim 1.

3. Drilling fluid according to either of the preceding claims, **characterized in that** the carboxylic acids are uronic acids or their salts, and the polysaccharides are pectins, alone or as a mixture with hemicelluloses.

4. Drilling fluid according to any one of claims 1 to 3, **characterized in that** the nanofibrils have a degree of crystallinity of less than 50%.

5. Drilling fluid according to any one of claims 1 to 4, **characterized in that** the nanofibrils have a cross section of between 2 nm and about 10 nm, and more particularly between 2 nm and about 4 nm.

6. Drilling fluid according to any one of claims 1 to 5, **characterized in that** the additive is carboxylated cellulose, preferably carboxymethylcellulose, with a degree of substitution of less than or equal to 0.95.

7. Drilling fluid according to any one of claims 1 to 5, **characterized in that** the additive is carboxylated cellulose, in salt or acid form, with a degree of substitution of greater than 0.95, a natural polysaccharide or a polyol, alone or in combination.

8. Drilling fluid according to any one of claims 1 to 6, **characterized in that** the co-additive is chosen from:
- saccharide monomers or oligomers,
- compounds of formula (R¹R²N)COA, in which formula R¹ or R², which may be identical or different, represent hydrogen or a C₁-C₁₀ and preferably C₁-C₅ alkyl radical, A represents hydrogen, a C₁-C₁₀ and preferably C₁-C₅ alkyl radical or a group R'¹R'²N with R'¹ and R'², which may be identical or different, representing hydrogen or a C₁-C₁₀ and preferably C₁-C₅ alkyl radical,
- cationic or amphoteric surfactants, alone or as a mixture.

9. Drilling fluid according to any one of claims 1 to 5 and 7, **characterized in that** the co-additive is chosen from one or more of the following compounds:
- carboxylated cellulose with a degree of substitution of less than or equal to 0.95, preferably carboxymethylcellulose,
- saccharide monomers or oligomers,
- compounds of formula (R¹R²N)COA, in which formula R¹ or R², which may be identical or different, represent hydrogen or a C₁-C₁₀ and preferably C₁-C₅ alkyl radical, A represents hydrogen, a C₁-C₁₀ and preferably C₁-C₅ alkyl radical or a group R'¹R'²N with R'¹ and R'², which may be identical or different, representing hydrogen or a C₁-C₁₀ and preferably C₁-C₅ alkyl radical,
- cationic or amphoteric surfactants.

10. Drilling fluid according to any one of claims 1 to 9, **characterized in that** the content of additive and optionally of co-additive is less than 30% by weight relative to the weight of nanofibrils and of additive and of co-additive.

11. Drilling fluid according to any one of the preceding claims, **characterized in that** the content of cellulose nanofibrils is between 0.05 and 2% relative to the total weight of fluid, preferably 0.05 to 1%.

12. Drilling fluid according to any one of the preceding claims, **characterized in that** the composition is obtained from a drying operation, the temperature of which should limit any degradation of the carboxylic acids, acidic polysaccharides, hemicelluloses and/or additives and co-additives.

13. Drilling fluid according to claim 12,
**characterized in that** the temperature is between 30°C and 80°C and preferably between 30°C and 60°C.

14. Drilling fluid according to any one of the preceding claims, **characterized in that** it comprises a filtrate-reducing agent, in an amount of between 0 and 1% relative to the total weight of the fluid.

15. Drilling fluid according to the preceding claim, **characterized in that** the filtrate-reducing agent is chosen from cellulosic compounds, polyacrylamides, high molecular weight polyacrylates, succinoglycans, native starch or its derivatives and charcoal, alone or in combination.

16. Drilling fluid according to any one of the preceding claims, **characterized in that** it comprises a thinning or dispersing agent in an amount of between 0 and 1% relative to the total weight of the fluid.

17. Drilling fluid according to the preceding claim, **characterized in that** the thinning or dispersing agent is chosen from polyphosphates, tannins, lignosulphonates, lignin derivatives, peats, lignites, polyacrylates and polynaphthalene sulphonates, alone or as a mixture.

18. Drilling fluid according to any one of the preceding claims, **characterized in that** it comprises an oxygen scavenger in a content of between 0 and 0.25% relative to the total weight of the fluid.

19. Drilling fluid according to one of the preceding claims, **characterized in that** it comprises a weighting compound chosen from alkaline-earth metal sulphates, carbonates and silicates, alkaline-earth metal or zinc bromides, and iron oxides.

20. Drilling fluid according to any one of the preceding claims, **characterized in that** it comprises at least one mineral colloid chosen from attapulgite, baryta and bentonite, alone or as a mixture.

21. Drilling fluid according to any one of the preceding claims, **characterized in that** it comprises salts.

22. Drilling fluid according to the preceding claim, **characterized in that** the salts are chosen from alkali metal halides, sulphates, carbonates, bicarbonates, silicates and phosphates, alone or as a mixture.

23. Drilling fluid according to either of claims 21 and 22, **characterized in that** the salt content is between 0.01 and 2% relative to the total weight of the fluid.
